# EUROPÄISCHE PATENTANMELDUNG

(11) **EP 1 078 598 A1**
(43) Veröffentlichungstag der Anmeldung: **28.02.2001**
(21) Anmeldenummer: 00118069.4
(22) Anmeldetag: 23.08.2000
(51) Int. Cl.: A61B 5/103

(54) **Verfahren und Einrichtung zur Klassifizierung von Hautanomalien, insbesondere von Melanomen**

(30) Priorität: 27.08.1999 DE 19940895
(71) Anmelder: Institut für Neurosimulation und Bildtechnologien GmbH, 39120 Magdeburg (DE)
(72) Erfinder: Albrecht, Peter, 39104 Magdeburg (DE); Michaelis, Bernd, Prof. Dr.-Ing. habil., 39175 Biederitz (DE)
(74) Vertreter: Leinung, Günter

(57) **Zusammenfassung**

Bösartige Melanome sind erst in ihrer Spätphase gut erkennbar, dann aber sehr gefährlich. In ihrer frühen Phase sind sie jedoch relativ einfach und erfolgreich behandelbar. Das Problem ist, dass sie in diesem Zeitabschnitt nur schwer identifizierbar sind; d.h. die Klassifizierung der Anomalien, insbesondere in bösartige oder gutartige Melanome, ist schwierig.

Ein Verfahren zur Klassifizierung ist dadurch gekennzeichnet, dass die physikalischen Parameter wie die Farbe, die Fläche sowie die dreidimensionale Form sowie deren zeitliche Änderungen messtechnisch erfasst, gespeichert, klassifiziert bzw. bewertet werden, wofür bevorzugt ein neuronales Netz verwendet wird.

Eine Einrichtung zur Klassifizierung besteht aus einem Messkopf, der einen Musterprojektor und zwei kalibrierte Kameras umfasst, wovon mindestens eine zur Aufnahme von Farbbildern geeignet ist.

## Beschreibung

Die Erfindung betrifft ein Verfahren und eine Einrichtung zur Klassifizierung von Hautanomalien, insbesondere von Melanomen und melanomähnlichen Erscheinungen.

Bösartige Melanome sind erst in ihrer Spätphase gut erkennbar, dann aber sehr gefährlich. In ihrer frühen Phase sind sie jedoch relativ einfach und erfolgreich behandelbar. Das Problem ist, dass sie in diesem Zeitabschnitt nur schwer identifizierbar sind; d. h. die Klassifizierung der Anomalien, insbesondere in bösartige oder gutartige Melanome, ist schwierig.

Die Ursache hierfür liegt darin, dass ein relativ großer Personenkreis bewertet werden muss. Aus Kapazitäts- und finanziellen Gründen haben die entsprechenden Personen in der Regel nur Kontakt zu ihrem Hausarzt, der bisher nicht über spezielle Apparate bzw. Spezialwissen verfügt, um eine korrekte Klassifizierung der Hautanomalien der Patienten, insbesondere der Melanome, vornehmen zu können.

Ziel der Klassifizierung ist die Zuordnung oder der Ausschluss des Patienten zu einer kritischen Gruppe. Bei einer Zuordnung erfolgt eine intensivere Beobachtung. Diese intensivere Beobachtung kann durch eine Telematikanwendung oder durch Überweisung zu einem Spezialisten erfolgen. Das Ergebnis der Entscheidung sollte auf alle Fälle darin bestehen, dass jede Person mit bösartigem Melanom zur kritischen Menge zugeordnet wird. In dieser Menge sind dann allerdings auch Personen mit gutartigen Melanomen. Ein untergeordnetes Ziel besteht somit in der Reduzierung jener Untergruppe, um die Methode zu effektivieren. Dabei darf das übergeordnete Ziel jedoch nicht 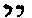 angetastet" werden.

Zur Optimierung der Klassifizierung werden erfindungsgemäß mit einem Verfahren und einer Einrichtung die wesentlichen physikalischen Parameter der Hautanomalien, insbesondere der Melanome und melanomähnlichen Erscheinungen sowie deren zeitliche Änderungen, messtechnisch umfassend erfasst, gespeichert und klassifiziert bzw. bewertet. Diese physikalischen Parameter sind: die Farbe, die Fläche sowie die dreidimensionale Form und somit auch das relative Wachstum.

Bisher wurden in der Regel nur die Farbe des Melanoms und die Änderung der Farbe beobachtet. Teilweise erfolgte auch eine Berücksichtigung der Fläche (Umfang, Durchmesser, Form).

Die wesentliche Schwachstelle bekannter Lösungen ist die Vernachlässigung der räumlichen Information. So kann z. B. ein relativ starkes Höhenwachstum des Melanoms eintreten, bei nur geringfügigen Farb- und Flächenänderungen. Somit bliebe ein aktives Melanom unentdeckt.

Demgegenüber wird durch die Erfindung auch die dreidimensionale Form der Hautanomalien, insbesondere der Melanome und melanomähnlichen Erscheinungen, durch ein genaues und absolut messendes Verfahren in den Klassiflzierungsprozess einbezogen.

Hierzu werden die dreidimensionale Form der Hautanomalien und alle weiteren gemessenen Parameter im Klassifizierungsprozess verknüpft und somit gemeinsam bewertet. Hierfür wird ein neuronales Netz verwendet.

Bestandteil der erfindungsgemäßen Einrichtung ist ein Messkopf, der aus einem kalibrierten Kamerapaar, nachfolgend als Stereokameras bezeichnet, und einem Musterprojektor besteht. Von diesen beiden Kameras ist mindestens eine zur Aufnahme von Farbbildern geeignet. Die Stereokameras sind so justiert, dass mit ihnen eine Fläche von ca. 3 x 3 m² vermessen werden kann. Unter Umständen ist eine dritte Kamera sinnvoll. Diese dient zur Aufnahme eines größeren Hautbereiches für die Bestimmung einer Referenzfarbe. Der Messkopf ist beweglich und kann per Hand oder automatisch an eine geeignete Stelle positioniert werden.

Um die notwendigen Bilder zur Bestimmung der Parameter der Hautanomalien zu gewinnen, eignet sich folgende Vorgehensweise:

Zunächst wird ein großflächiger Hautbereich mit mindestens einer Farbkamera aufgenommen. Das entstandene Bild wird für eine Farbkorrektur der aktuellen Melanomfarbe benötigt.

Anschließend wird der Messkopf so positioniert, dass das Melanom einen großen Teil beider Kamerabilder abdeckt (Nahbereich). Nun kann mindestens ein weiteres Farbbild (sogenanntes Hellbild, da kein Muster mit dem Projektor aufprojiziert wird) aufgenommen werden, aus dem die aktuelle Farbe des Melanoms bestimmt wird.

Des weiteren wird bei unveränderter Lage des Messkopfes durch die Stereokameras ein Bildpaar augenommen. Hierbei wird in der Regel ein geeignetes Muster, das verfahrensbedingt benötigt wird, mit Hilfe des Projektors auf die Messfläche projiziert. Dieses Bildpaar ist Grundlage der dreidimensionalen Berechnung der Oberfläche der Hautanomalie, insbesondere des Melanoms.

Grundlage der Bestimmung der örtlichen Farbwerte ist ein Kamerabild aus dem Nahbereich. Diese Farbwerte werden durch die Aktivität der Hautanomalie, insbesondere des Melanoms, aber auch durch den jahreszeitlich bedingten Bräunungsgrad, beeinflusst. Um letztgenannten Einfluss zu minimieren, ist eine Farbkorrektur sinnvoll, für die das Bild eines großflächigen Hautbereiches verwendet wird, der außerhalb der Hautanomalie liegt. Diese Korrektur kann auf klassische Weise (Verwendung eines Offsets und einer Helligkeitskorrektur) oder durch ein neuronales Netz erfolgen.

Die Bestimmung der dreidimensionalen Form erfolgt ausgehend von einem Bildpaar oder einer Folge von Bildpaaren der Stereokameras. Für die Berechnung werden bekannte Algorithmen aus der Bildverarbeitung verwendet. Diese basieren auf der Bestimmung korrespondierender Punktepaare durch Matchingverfahren, basierend auf Korrelationsalgorithmen.

Des weiteren ist es nun möglich, die gewonnenen Farbwerte dem 3D-Oberflächenmodell zuzuordnen, da die entsprechenden Bilder aus derselben Position aufgenommen wurden.

Die auf diese Weise bestimmten Parameter der Hautanomalie, insbesondere des Melanoms, sind Bestandteil der Eingangsdaten für ein neuronales Netz. Weitere Eingangsdaten sind sonografische Messdaten und patiententypische Daten wie Veranlagung, Lebensraum, Lebensweise usw. Diese Eingangsdaten werden durch ein neuronales Netz bewertet. Erfolgt die Identifizierung als kritischer Fall, dann ist eine intensivere Beobachtung notwendig.

In der Regel ist in so einem Fall ein Vergleich mit bereits durch Spezialisten bewerteten ähnlichen Bildern und weiteren Daten einer Datenbank sinnvoll. Diese Datenbank befindet sich an einer zentralen Stelle. Die Übertragung der Daten kann somit durch eine Telematikanwendung realisiert werden.

Die Auswahl der Daten erfolgt durch einen indexierten Zugriff, d. h. aus den aufgenommenen Bildern werden Merkmale abgeleitet, auf deren Grundlage aus der Datenbank geeignete Bilder und Daten mit ähnlichen Eigenschaften ausgewählt werden. Hierdurch kann eine Gegenüberstellung mit vergleichbaren Eigenschaften ausgewählt werden. Dieses Vergleichsmaterial wurde bezüglich des Charakters von Spezialisten bewertet (bösartig oder gutartig).

Somit kann durch diese Vorgehensweise der Klassifizierungsprozess weiter verbessert werden. Der Kreis mit möglicherweise bösartigen Melanomen wird reduziert. Die weiterhin in Frage kommenden Personen können nun an einen Spezialisten überwiesen werden.

Weitere mögliche Anwendungen der Erfindung sind die Vermessung und Bewertung von:
- Wunden. Es werden die Wundgröße, das Wundvolumen sowie die Farbwerte zur Einschätzung des Heilungsvorganges bestimmt.
- Testen der Störungsempfindlichkeit. Messung der Farbänderung von bestrahlter Haut.
- Messung der Farbänderung und der Höhenänderung der Haut bei einem Allergietest.
- Falten.
- Flechten.

## Patentansprüche

1. Verfahren zur Klassifizierung von Hautanomalien, insbesondere von Melanomen und melanomähnlichen Erscheinungen, bei dem deren wesentliche physikalische Parameter wie die Farbe, die Fläche sowie die dreidimensionale Form sowie deren zeitliche Änderungen messtechnisch umfassend erfasst, gespeichert und klassifiziert bzw. bewertet werden, indem die dreidimensionale Form der Hautanomalien und alle weiteren gemessenen Parameter im Klassifizierungsprozess verknüpft und somit gemeinsam bewertet werden, wofür bevorzugt ein neuronales Netz verwendet wird.

2. Einrichtung zur Klassifizierung von Hautanomalien, insbesondere von Melanomen und melanomähnlichen Erscheinungen, mit einem Messkopf, der aus mindestens zwei kalibrierten Kameras, nachfolgend als Stereokameras bezeichnet, und einem Musterprojektor besteht, wobei von den beiden Kameras mindestens eine zur Aufnahme von Farbbildern geeignet ist und gegebenenfalls eine dritte Kamera zur Aufnahme eines größeren Hautbereiches für die Bestimmung einer Referenzfarbe vorgesehen ist.
